(19) Europäisches Patentamt

European Patent Office

Office européen des brevets

(11) Veröffentlichungsnummer: **0 563 176 B1**

(12) **EUROPÄISCHE PATENTSCHRIFT**

(45) Veröffentlichungstag der Patentschrift: **04.01.95**

(21) Anmeldenummer: **92901683.0**

(22) Anmeldetag: **18.12.91**

(86) Internationale Anmeldenummer:
**PCT/DE91/01002**

(87) Internationale Veröffentlichungsnummer:
**WO 92/11000 (09.07.92 92/17)**

(51) Int. Cl.⁶: **A61K 9/16**

(54) **VERFAHREN ZUR HERSTELLUNG WIRKSTOFFHALTIGER MIKROPARTIKEL AUS HYDROLYTISCH ABBAUBAREN POLYMEREN.**

(30) Priorität: **22.12.90 DE 4041563**

(43) Veröffentlichungstag der Anmeldung:
**06.10.93 Patentblatt 93/40**

(45) Bekanntmachung des Hinweises auf die
Patenterteilung:
**04.01.95 Patentblatt 95/01**

(84) Benannte Vertragsstaaten:
**AT BE CH DE DK ES FR GB GR IT LI LU MC NL SE**

(56) Entgegenhaltungen:
**EP-A- 0 275 961      EP-A- 0 315 875**
**EP-A- 0 322 687      EP-A- 0 350 246**
**EP-A- 0 421 557      WO-A-91/09079**
**CH-A- 666 406**

(73) Patentinhaber: **SCHWARZ PHARMA AG**
**Alfred-Nobel-Strasse 10**
**D-40789 Monheim (DE)**

(72) Erfinder: **BISKUP, Heike**
**Akazienallee 13**
**D-4018 Langenfeld (DE)**
Erfinder: **GORISSEN, Elke**
**Turnhallenstrasse 15**
**D-7600 Offenburg (DE)**
Erfinder: **SCHNEIDER, Hannelore**
**Ulmenstrasse 45**
**D-4000 Düsseldorf (DE)**

EP 0 563 176 B1

**Beschreibung**

Die vorliegende Erfindung betrifft ein Verfahren zur Herstellung wirkstoffhaltiger Mikropartikel aus hydrolytisch abbaubaren Polymeren.

Aus zahlreichen Publikationen ist bekannt, daß resorbierbare Polyester, insbesondere solche auf der Basis von Milch- oder Glykolsäure, insbesondere deren Copolymere, im menschlichen oder tierischen Gewebe oder in menschlichen oder tierischen Flüssigkeiten vollständig zu körpereigenen Verbindungen abgebaut werden, wobei die Abbaurate der Polymere, je nach Verwendungszweck, von wenigen Stunden bis zu mehreren Monaten variiert werden kann.

Die Abbauprodukte gelangen in den normalen biochemischen Stoffwechsel und werden entweder direkt ausgeschieden oder letztlich zu Wasser und Kohlendioxid metabolisiert.

Von besonderem Interesse und Bedeutung sind die Anwendungen resorbierbarer Polyester in galenischen Zubereitungen mit verzögerter Wirkstofffreigabe zur Herstellung von Depotformen.

Derartige Polyester können jedoch im menschlichen oder tierischen Organismus nur dann verwendet werden, wenn sie frei von Verunreinigungen sind, die möglicherweise Irritationen auslösen können. Solche Verunreinigungen sind beispielsweise nicht umgesetzte Restmonomere, Molekulargewichtsregulatoren oder Polymerisationskatalysatoren.

In der DE-OS 37 08 916 ist der Stand der Technik hierzu dargestellt.

Unter Verwendung derartiger resorbierbarer Polyester hergestellte Retardarzneiformen, die geeignet sind als subcutane Injektionen oder Implantationen in den Körper eingebracht zu werden, wurden bislang nach folgenden Verfahren hergestellt:

- Mikroverkapselung mit organischen Lösungsmitteln
  (L.M. Sanders et al., J. Contr. Release, 2 (1985) 187 oder P.B. Deasy, Microencapsulation and Related Drug Processes, M. Dekker Inc., New York 1984);
- Emulgierung und anschließende Lösungsmittelverdampfung
  (T.R. Tice & R.M. Gilley, J. Contr. Release, 2 (1985) 343);
- Sprühtrocknung
  (D.L. Wise et al., Life Sci., 19 (1976) 867;
- Extrusion
  (A.J. Schwope et al., Life Sci., 17 (1975) 1877);
- Schmelzeinbettung
  (A.J. Schwope et al., Life Sci., 17 (1975) 1877); oder
- Grenzflächenpolymerisation
  (G. Birrenbach & P. Speiser, J. Pharm. Sci., 65 (1976) 1763);

Die genannten Verfahren sind jedoch entweder mit dem Nachteil behaftet, daß sie mit großen Mengen toxischer organischer Lösungsmittel arbeiten, wobei die resultierenden Retardarzneiformen in Form von Polymereinbettungen hohe Lösungsmittelrestkonzentrationen aufweisen; vgl. J.P. Benoit et al., Int. J. Pharmaceutics, 29 (1986) 95, oder die genannten Verfahren arbeiten bei hohen Temperaturen oder Drücken, die insbesondere zu hohen lokalen Temperaturerhöhungen führen und die inkorporierten Arzneistoffe schädigen können; vgl. L.M. Sanders et al., J. Pharm. Sci., 75 (1986) 356. Wenn eine solche Arzneiform über längere Zeit unter der Haut oder im Gewebe verbleibt, sind von organischen Lösungsmitteln lokal toxische Gewebereaktionen zu erwarten. Daher müssen Lösungsmittelrestmengen soweit als möglich aus den erwähnten Produkten entfernt werden.

Eine detaillierte Darstellung der oben genannten, zum Stand der Technik gehörenden Herstellverfahren findet sich in der DE-OS 37 44 329.

Schließlich ist ein als "Aerosol Solvent Extraktion System" (ASES) bezeichnetes Verfahren zur Herstellung wirkstoffbeladener Mikropartikel aus der EP 0 322 687 A2 bekannt.

Mit diesem Verfahren werden wirkstoffbeladene Mikropartikel mit Hilfe fluider Gase hergestellt. In einer überkritischen Atmosphäre werden aus einer Lösung von Polymeren und Wirkstoff Mikropartikel geformt, wobei das Lösungsmittel durch Aufnahme in die Gasphase entfernt wird.

Obwohl dieses Verfahren geeignet ist, wirkstoffbeladene Mikropartikel mit einem Mindestmaß an organischen Lösungsmitteln (unter 10 ppm) ohne Restmonomere, Molekulargewichtsregulatoren oder Polymerisationskatalysatoren auch autosteril herzustellen, ist jedoch nachteilig, daß bei Batch-Vergrößerungen keine zuverlässige konstante Teilchengrößenverteilung zu erzielen ist und nicht alle hydrolytisch abbaubaren Polymere zuverlässig zu Mikropartikeln verarbeitet werden können.

Aufgabe der vorliegenden Erfindung ist es daher, wirkstoffbeladene Mikropartikel mit gleichmäßiger Teilchengrößenverteilung aus hydrolytisch abbaubaren Polymeren, insbesondere aus Copolymeren der Milch- und Glykolsäure, herzustellen.

Diese Aufgabe wurde dadurch gelöst, daß jeweils mindestens eine physiologisch verträgliche Aminosäure einer Lösung aus hydrolytisch abbaubaren Polymeren oder Copolymeren und mindestens einem Wirkstoff in entsprechenden Lösungsmitteln zugesetzt wird, in einer überkritischen Atmosphäre Mikropartikel geformt werden, wobei die Lösungsmittel durch Aufnahme in die Gasphase entfernt werden.

Erfindungsgemäß kann jeder Wirkstoff eingesetzt werden. Beispiele für Wirkstoffe sind Arzneimittel, Toxine, Viren. Zum Begriff des Arzneimittels wird auf die US-PS 3,773,919 verwiesen.

Alle biologisch verträglichen, hydrolytisch abbaubaren Polymere und Copolymere können als Trägerstoffe verwendet werden.

Als Beispiele seien genannt:

Poly-l-lactid (l-PLA), Poly-d,l-lactid (PLA), Poly-l-lactid-coglycolide (l-PLGA) sowie Poly-d,l-lactid-coglycolide (PLGA) mit variablen Anteilen der Monomere von Milchsäure (PLA) und Glycolsäure (GA). Vorzugsweise umfaßt das Polymer Lactidcoglycolid ein Molverhältnis zwischen 85:15 und 50:50, besonders bevorzugt ist das Molverhältnis 75:25.

Erfindungsgemäß verwendbare Aminosäuren, ohne darauf beschränkt zu sein, sind L-Lysin, L-Phenylalanin, L-Tryptophan oder D,L-Phenylalanin.

Nach einer Ausbildung der Erfindung werden als fluides Gas Distickstoffoxid, Kohlendioxid, Halogenkohlenwasserstoffe, gesättigte oder ungesättigte Kohlenwasserstoffe, Distickstoffdioxid oder Ammoniak oder Mischungen davon verwendet.

Nach einer anderen Ausbildung der Erfindung sind auch entsprechend geeignete Gase sowie niedrig siedende Flüssigkeiten und Mischungen davon, die sich in einen überkritischen Zustand überführen lassen, jeweils als fluides Gas verwendbar.

Die folgenden Ausführungsbeispiele erläutern die Erfindung.

Ausführungsbeispiel 1

Herstellung von Buserelin enthaltenden Mikropartikeln
Polymer: Poly-(D,L)lactidcoglycolid, i.V. 0,8 dl/g

0,375 g L-Lysin (Aldrich) werden in 25 ml Eisessig (Merck) gelöst; 2,5 g Poly-(D,L)lactidcoglycolid (Monomerenverhältnis 75:25) werden in 75 ml Dichlormethan (Merck) gelöst. Beide Lösungen werden vereinigt. Dann werden 0,06 g Buserelin zugegeben und gerührt, bis eine klare Lösung entstanden ist; 0,06 %-ige Lösung, bezogen auf Buserelin.

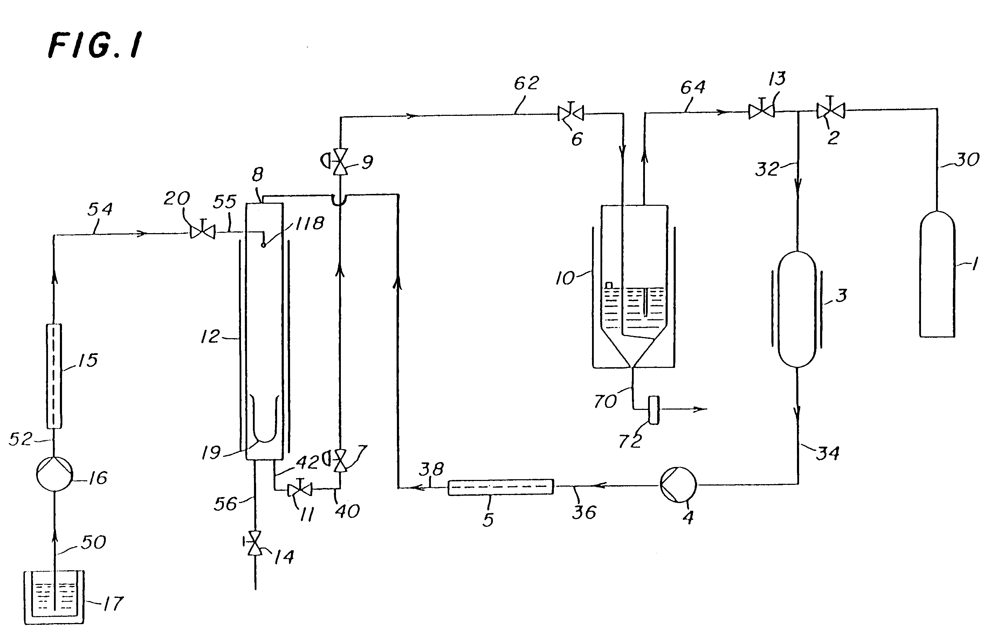

Diese Lösung wird in ein Reservoir (17), - siehe FIG. 1 -, gegeben, mittels Leitung (50) einer Kolbenhubpumpe (16) zugeführt, nach Durchströmen von Leitung (52), eines Wärmetauschers (15) Leitung (54) einem Ventil (20) und schließlich über Leitung (55) der Düse (118) zugeleitet. Mit einem Überdruck von 94-96 bar wird die Buserelin und Copolymer enthaltende Lösung durch eine konventionelle Einstoffdüse (118), Typ Schlick 121 V, in die Kolonne (12) versprüht, wobei $CO_2$ im überkritischen Zustand mit 90 bar/36°C und einem Durchsatz von 8,9 kg/h im Gleichstrom, über Einlaß (8), durch die Kolonne geleitet wird. Die Düse hat einen Durchmesser von 0,5 mm, der Sprühwinkel beträgt 10°.

Entsprechend der hohen Affinität des überkritischen $CO_2$ zum Lösungsmittel wird den primär gebildeten Tröpfchen Lösungsmittel entzogen; zurück bleiben kugelförmige feste Lösungen. Das mit dem Lösemittel beladene $CO_2$ entströmt durch Leitungen (42) und (40), gesteuert durch 2 Magnetventile (7 und 9), dem Kolonnenende und wird auf 60 bar entspannt. Die Ventile sind so geschaltet, daß die pro Zeiteinheit in die Kolonne einströmende Menge des fluiden Gases unter Aufrechterhaltung des Arbeitsdruckes der Kolonne entströmen kann.

Das durch die Entspannung auf 60 bar abgekühlte, mit Lösungsmittel beladene $CO_2$ wird mittels Leitung (62) in den auf 21°C geheizten Separator (10) geleitet, wo sich das Lösungsmittelgemisch infolge der unter diesen Bedingungen stark verminderten Löslichkeit im $CO_2$ abscheidet. Das vom Lösungsmittelgemisch befreite $CO_2$ wird mittels Leitungen (64 und 32) durch Druck- und Temperaturerhöhung erneut in den überkritischen Zustand überführt (90 bar, 36°C) und zur weiteren Trocknung der entstandenen Partikel erneut der Kolonne über Leitung (34), Flüssiggaspumpe (4), Leitung (36), Wärmetauscher (5), Leitung (38) durch Einlaß (8) zugeführt.

Die Entnahme des im Separator (10) abgetrennten Lösungsmittelgemischs erfolgt nach Abtrennung des Separators (10) aus dem Kreislauf durch Ventile (6) und (13) und Entspannung auf Atmosphärendruck.

Nach Beendigung der eigentlichen Sprühzeit, die zwischen 20 und 50 min. beträgt, wird noch solange $CO_2$ durch die Kolonne geleitet, bis im Separator (10) kein Lösungsmittel mehr zurückgewonnen werden kann.

Nach Abschluß des Trocknungsvorganges wird der $CO_2$-Strom zur Kolonne abgestellt, die Kolonne über die Ventile (11) und (14) auf Atmosphärendruck entspannt und die Partikel am unteren Kolonnenende (19) entnommen.

Das aus der Kolonne abgenommene trockene Pulver besteht aus Buserelin-haltigen Kugeln mit einem Durchmesser von 5-10 $\mu$m.

Ausführungsbeispiel 2

Herstellung von LH-RH-Antagonist enthaltenden Mikropartikeln
Polymer: Poly-(D,L)lactidcoglycolid, i.V. 0,8 dl/g

Die Herstellung erfolgt analog Ausführungsbeispiel 1.

0,375 g L-Lysin (Aldrich), 0,06 g LH-RH-Antagonist, 2,5 g Poly(D,L-)lactidcoglycolid [75:25] i.V. 0,8 dl/g werden gemeinsam in 25 ml Eisessig (Merck) und 75 ml Dichlormethan (Merck) gelöst und solange gerührt, bis eine klare Lösung entstanden ist.

Diese Lösung wird mit 94-96 bar Überdruck in der Kolonne der Hochdruckanlage versprüht. Dabei wird $CO_2$ mit 90 bar/36°C im Gleichstrom durch die Kolonne geleitet. Der $CO_2$-Durchsatz beträgt 8,9 kg/h. Als Düse dient eine konventionelle Einstoffdüse vom Typ Schlick 121 V, mit einem Düsendurchmesser von 0,5 mm und einem Sprühwinkel von 10°.

Nach Beendigung der eigentlichen Sprühzeit wird solange $CO_2$ durch die Kolonne geleitet, bis im Separator der Hochdruckanlage kein Lösungsmittel mehr zurückgewonnen werden kann.

Das aus der Kolonne erhaltene trockene Pulver besteht aus Kugeln mit einem Durchmesser von 5-10 $\mu$m.

Ausführungsbeispiel 3

Herstellung von wirkstofffreien Mikropartikeln
Polymer: Poly-(D,L)lactidcoglycolid, i.V. 0,8 dl/g

Die Herstellung erfolgt analog Ausführungsbeispiel 1.

Eine Lösung aus 2,5 g Poly-(D,L)lactidcoglycolid) [75:25] i.V. 0,8 dl/g in 75 ml Dichlormethan und 25 ml Eisessig (jeweils Merck), die 0,375 g L-Lysin (Aldrich) enthält, wird mit ca. 94-96 bar Überdruck in der Kolonne der Hochdruckanlage versprüht. Gleichzeitig wird $CO_2$ mit 90 bar/36°C im Gleichstrom durch die Kolonne geleitet. Der $CO_2$-Durchsatz beträgt 8,9 kg/h. Als Düse dient eine konventionelle Einstoffdüse vom Typ Schlick 121 V mit einem Durchmesser von 0,5 mm und einem Sprühwinkel von 10°.

Nach Beendigung der eigentlichen Sprühzeit wird solange $CO_2$ durch die Kolonne geleitet, bis im Separator der Hochdruckanlage kein Lösungsmittel mehr zurückgewonnen werden kann.

Das aus der Kolonne erhaltene trockene Pulver besteht aus Kugeln mit einem Durchmesser von 5-10 $\mu$m.

Die folgende Tabelle 1 zeigt zusammenfassend weitere Beispiele einsetzbarer Polymere zur Herstellung wirkstoffhaltiger Mikropartikel.

Die Herstellung derartiger Mikropartikel erfolgt in gleicher Weise, wie in den Ausführungsbeispielen 1 bis 3 erläutert wird.

Tabelle 1

| Nr. | Polymer | inhärente Viscosität dl/g | Molverhältnis Lactid:Glycolid | Polymer (g) | Wirkstoff (g) | Aminosäure | Aminosäure (g) |
|---|---|---|---|---|---|---|---|
| 1 | Poly-(D,L)-lactid | 0,3 | | 2,0 | 0,06 | L-Lysin | 0,5 |
| 2 | Poly-(D,L)-lactid-coglycolid | 0,8 | 75:25 | 1,0 | 0,03 | L-Phenyl-alanin | 0,25 |
| 3 | Poly-(D,L)-lactid-coglycolid | 0,8 | 75:25 | 2,0 | 0,06 | D,L-Phenyl-alanin | 0,5 |
| 4 | Poly-(D,L)-lactid-coglycolid | 0,8 | 75:25 | 2,2 | 0,06 | L-Tryptophan | 0,55 |
| 5 | Poly-L-lactid | 0,56 | | 6,0 | 0,18 | L-Lysin | 0,9 |
| 6 | Poly-L-lactid | 1,0 | | 3,0 | 0,10 | L-Lysin | 0,45 |

Die Polymere werden jeweils in 75 ml Dichlormethan (Merck) und 25 ml Eisessig gelöst. Die jeweilige Menge Aminosäure wird zugegeben. D,L-Phenylalanin ist geeigneterweise in bis zu 50 ml Ethanol (Merck) vorzulösen.

Die erhaltenen Mikropartikel sind steril und frei von Lösemittelrückständen, Polymerisationskatalysatoren oder Initiatormolekülen.

Sie besitzen eine gleichmäßige Teilchengrößenverteilung von 5 bis 10 $\mu$m.

Sie können daher als Retardarzneiform für subcutane Injektionen oder Implantationen in den Körper Verwendung finden.

5

**Patentansprüche**

1. Verfahren zur Herstellung wirkstoffbeladener Mikropartikel aus hydrolytisch abbaubaren Polymeren mittels fluider Gase, dadurch gekennzeichnet, daß ein flüssiges Medium, enthaltend mindestens einen Wirkstoff, mindestens ein Polymer als Träger und mindestens eine Aminosäure mit einem fluiden Gas in Berührung gebracht wird, das flüssige Medium durch das fluide Gas entfernt wird und die vom flüssigen Medium befreite Wirkstoff-/Polymer-Trägerzubereitung in Form von Mikropartikeln gewonnen wird.

2. Verfahren zur Herstellung wirkstoffbeladener Mikropartikel aus hydrolytisch abbaubaren Polymeren mittels fluider Gase nach Anspruch 1, dadurch gekennzeichnet, daß die Polymere Copolymere sind.

3. Verfahren zur Herstellung wirkstoffbeladener Mikropartikel aus hydrolytisch abbaubaren Polymeren mittels fluider Gase nach Ansprüchen 1 und 2, dadurch gekennzeichnet, daß die Polymere Poly-l-lactid, Poly-d,l-lactid oder Poly-l-lactid-coglycolide oder Poly-d,l-lactidcoglycolide mit variablen Anteilen der jeweiligen Monomerenbestandteile sind.

4. Verfahren zur Herstellung wirkstoffbeladener Mikropartikel aus hydrolytisch abbaubaren Polymeren mittels fluider Gase nach Ansprüchen 1 bis 3, dadurch gekennzeichnet, daß das Polymer Lactidcoglycolid ein Molverhältnis zwischen 85:15 und 50:50, insbesondere 75:25 umfaßt.

5. Verfahren zur Herstellung wirkstoffbeladener Mikropartikel aus hydrolytisch abbaubaren Polymeren mittels fluider Gase nach Ansprüchen 1 bis 4, dadurch gekennzeichnet, daß die Aminosäure L-Lysin, L-Phenylalanin oder L-Tryptophan ist.

6. Verfahren zur Herstellung wirkstoffbeladener Mikropartikel aus hydrolytisch abbaubaren Polymeren mittels fluider Gase nach Ansprüchen 1 bis 5, dadurch gekennzeichnet, daß die Aminosäure D,L-Phenylalanin ist.

7. Verfahren zur Herstellung wirkstoffbeladener Mikropartikel aus hydrolytisch abbaubaren Polymeren mittels fluider Gase nach Ansprüchen 1 bis 6, dadurch gekennzeichnet, daß als fluides Gas Distickstoffoxid, Kohlendioxid, Halogenkohlenwasserstoffe, gesättigte oder ungesättigte Kohlenwasserstoffe, Distickstoffdioxid oder Ammoniak oder Mischungen davon verwendet werden.

8. Verfahren zur Herstellung wirkstoffbeladener Mikropartikel aus hydrolytisch abbaubaren Polymeren mittels fluider Gase nach Ansprüchen 1 bis 7, dadurch gekennzeichnet, daß geeignete Gase sowie niedrig siedende Flüssigkeiten und Mischungen davon, die sich in einen überkritischen Zustand überführen lassen, als fluides Gas verwendet werden.

**Claims**

1. A process for the manufacture of active substance-loaded microparticles from hydrolytically decomposable polymers by means of fluid gases, characterized in that a fluid medium containing at least one active substance, at least one polymer as a carrier and at least one amino acid, is brought in contact with a fluid gas, the fluid medium is removed by the fluid gas, and the active substance/polymer carrier preparation, which has been freed from the fluid medium, is recovered in the form of microparticles.

2. A process according to claim 1, characterized in that said polymers are copolymers.

3. A process according to claim 1, characterized in that said polymers are poly-l-lactide, poly-d,l-lactide or poly-l-lactide-coglycolides or poly-d,l-lactide-coglycolides with variable portions of the respective monomer components.

4. A process according to claim 3, characterized in that the polymer lactide-coglycolide comprises a molar ratio between 85:15 to 50:50.

5. A process according to claim 1, characterized in that the amino acid is L-lysine, L-phenylalanine, or L-tryptophane.

6. Process for the manufacture of active substance-loaded microparticles from hydrolytically decomposable polymers by means of fluid gases according to claim 1, characterized in that the amino acid is D,L-phenylalanine.

7. Process for the manufacture of active substance-loaded microparticles from hydrolytically decomposable polymers by means of fluid gases according to claim 1, characterized in that dinitrogen oxide, carbon dioxide, halogenated hydrocarbons, saturated or unsaturated hydrocarbons, dinitrogen dioxide, or ammonia, or mixtures thereof, are used as a fluid gas.

8. Process for the manufacture of active substance-loaded microparticles from hydrolytically decomposable polymers by means of fluid gases according to claim 1, characterized in that suitable gases as well as low-boiling liquids and mixtures thereof, which can be converted into a supercritical condition, are used as a fluid gas.

**Revendications**

1. Procédé de fabrication de microparticules chargées de principes actifs à partir de polymères dégradables par voie hydrolytique, à l'aide de gaz fluides, caractérisé en ce que l'on met en contact un milieu liquide, contenant au moins un principe actif, au moins un polymère servant de support ou véhicule et au moins un aminoacide avec un gaz fluide, le milieu liquide est enlevé par le gaz fluide et on récupère la préparation à support ou véhicule de polymère/principe actif, débarrassée du milieu liquide, sous forme de microparticules.

2. Procédé de fabrication de microparticules chargées de principes actifs à partir de polymères dégradables par voie hydrolytique, à l'aide de gaz fluides, suivant la revendication 1, caractérisé en ce que les polymères sont des copolymères.

3. Procédé de fabrication de microparticules chargées de principes actifs à partir de polymères dégradables par voie hydrolytique, à l'aide de gaz fluides, suivant les revendications 1 et 2, caractérisé en ce que les polymères sont le poly-l-lactide, le poly-d,l-lactide, ou le poly-l-lactidecoglycolide, ou des poly-d,l-lactidecoglycolides avec des fractions variables des constituants monomériques concernés.

4. Procédé de fabrication de microparticules chargées de principes actifs à partir de polymères dégradables par voie hydrolytique, à l'aide de gaz fluides, suivant les revendications 1 à 3, caractérisé en ce que le polymère est le lactidecoglycolide d'un rapport molaire compris entre 85:15 et 50:50, plus particulièrement de 75:25.

5. Procédé de fabrication de microparticules chargées de principes actifs à partir de polymères dégradables par voie hydrolytique, à l'aide de gaz fluides, suivant les revendications 1 à 4, caractérisé en ce que l'aminoacide est la L-lysine, la L-phénylalanine, ou le L-tryptophane.

6. Procédé de fabrication de microparticules chargées de principes actifs à partir de polymères dégradables par voie hydrolytique, à l'aide de gaz fluides, suivant les revendications 1 à 5, caractérisé en ce que l'aminoacide est la D,L-phénylalanine.

7. Procédé de fabrication de microparticules chargées de principes actifs à partir de polymères dégradables par voie hydrolytique, à l'aide de gaz fluides, suivant les revendications 1 à 6, caractérisé en ce qu'à titre de gaz fluide, on utilise l'oxyde azoteux ou protoxyde d'azote, le dioxyde de carbone ou anhydride carbonique, des hydrocarbures halogénés, des hydrocarbures saturés ou insaturés, le dioxyde de diazote, ou l'ammoniac, ou leurs mélanges.

8. Procédé de fabrication de microparticules chargées de principes actifs à partir de polymères dégradables par voie hydrolytique, à l'aide de gaz fluides, suivant les revendications 1 à 7, caractérisé en ce que l'on utilise, à titre de gaz fluide, des gaz appropriés, ainsi que des liquides à bas points d'ébullition et des mélanges de ceux-ci qui se laissent amener dans un état supercritique.

# FIG. 1

EP 0 563 176 B1